# EUROPÄISCHE PATENTSCHRIFT

(11) **EP 2 535 650 B1**
(45) Veröffentlichungstag und Bekanntmachung des Hinweises auf die Patenterteilung: **14.12.2016**
(21) Anmeldenummer: 12179711.2
(22) Anmeldetag: 13.01.2010
(51) Int. Cl.: F24F 3/16, A61L 2/20, A61L 9/16, B08B 15/02, B01D 46/00, A61L 9/015

(54) **Dekontaminationsanordnung sowie Verfahren**
Decontamination arrangement and method
Agencement de décontamination ainsi que procédé

(43) Veröffentlichungstag der Anmeldung: 19.12.2012
(62) Teilanmeldung aus: 10702591.8
(73) Patentinhaber: Metall + Plastic GmbH, 78315 Radolfzell (DE)
(72) Erfinder: von Stenglin, Christoph, 78315 Radolfzell (DE); Hauk, Jürgen, 85354 Freising (DE)
(74) Vertreter: Wagner, Kilian

(56) Entgegenhaltungen:
- EP-A1- 0 604 925
- EP-A1- 1 426 122
- WO-A1-98/50134
- WO-A1-2005/113169
- US-A- 6 010 400

## Beschreibung

Die Erfindung betrifft eine Dekontaminationsanordnung (Dekontaminationsvorrichtung) gemäß dem Oberbegriff des Anspruchs 1, insbesondere für pharmatechnische Anwendungen, umfassend einen zu dekontaminierenden Raum, insbesondere einen Isolatorraum, sowie eine zum Entziehen von Gas- und/oder dampfförmigen Dekontaminationsmittel, insbesondere Wasserstoffperoxid, aus der Raumluft ausgebildete Reinigungseinrichtung. Ferner betrifft die Erfindung ein Verfahren zum Entfernen von Gas- und/oder dampfförmigen Dekontaminationsmittel, insbesondere von Wasserstoffperoxiddampf, aus einem zu dekontaminierenden Raum, insbesondere mittels einer wie zuvor beschreiben ausgebildeten Dekontaminationsanordnung. Für den Fall der Inkludierung des Verfahrensschrittes der Zuführung von Dekontaminationsmittel in dem zu dekontaminierenden Raum zeitlich vor dem Freispülschritt handelt es sich um ein kombiniertes Dekontaminations-/Dekontaminationsmittelentfernungsverfahren.

Aus der EP 0 604 925 B1 ist ein Isolator für pharmatechnische Anwendungen bekannt. Dieser umfasst einen zu dekontaminierenden Manipulatorraum, über welchem ein sogenannter Umlufterzeugerraum, umfassend ein Umluftgebläse, angeordnet ist. Der zu dekontaminierende Raum ist mit Doppelglasscheiben ausgestattet, zwischen denen ein Strömungskanal ausgebildet ist, der es ermöglicht, dass die Raumluft während der Dekontamination zwischen dem Manipulatorraum und dem Umluftraum mit dem Umluftgebläse zirkuliert werden kann. Zur Dekontamination wird üblicherweise Wasserstoffperoxiddampf eingesetzt, der mittels eines Wasserstoffperoxidverdampfers erzeugt wird, welcher üblicherweise in einer Leitung angeordnet ist, die aus dem zu dekontaminierenden Raum ausmündet, um die mit Dekontaminationsmitteldampf anzureichernde Raumluft ansaugen zu können und in einem Bereich nach dem Verdampfer, die wieder in den zu dekontaminierenden Raum einmündet, um die mit Dekontaminationsmittel angereicherte Luft wieder in den zu dekontaminierenden Raum abgeben zu können.

Die zuvor beschrieben ausgebildeten Dekontaminationseinrichtungen weisen einen Frischluftanschluss auf, über den konditionierte (getrocknete und temperierte) Frischluft zugeführt werden kann, um das Dekontaminationsmittel aus dem zu dekontaminierenden Raum nach erfolgter Dekontamination ausblasen, d.h. den Raum spülen zu können. Dieses sogenannte Freispülen nimmt einen erheblichen Zeitraum in Anspruch, der in Abhängigkeit der Größe des Isolators und der gewünschten, zu erzielenden (minimalen) Dekontaminationsmittelkonzentration von beispielsweise 1 ppm oder weniger drei bis zehn Stunden betragen kann. Die in der Praxis zum Einsatz kommenden Frischluftkonditioniereinrichtungen umfassen ein Zuluftgebläse (Frischluftgebläse), welches üblicherweise derart ausgelegt ist, dass mit diesem in den zu dekontaminierenden Raum und allen damit strömungstechnisch verbundenen Räumen ein etwa 150facher Luftwechsel pro Stunde realisierbar ist. Es ist also etwa ein 150faches Raumvolumen pro Stunde mittels des Frischluftgebläses zuführbar.

Häufig sind bekannte Dekontaminationsanordnungen im Abluftkanal mit einer katalytisch wirkenden Reinigungseinrichtung versehen, die die in die Umgebung abgelassene Raumluft während des Freispülens des zu dekontaminierenden Raumes zum Schutze der Umwelt von Dekontaminationsmittel befreit. Die Reinigungseinrichtung besteht dabei im Wesentlichen aus einem Katalysator, welcher wiederum aus als Schüttgut vorliegenden MnO₂-Kugeln besteht.

Nachteilig bei den bekannten Dekontaminationsanordnungen ist die lange Freispülzeit (Reinigungszeit), die benötigt wird, um die Dekontaminationsmittelkonzentration in der Raumluft auf das gewünschte Maß zu reduzieren.

Aus der US 6,010,400 A ist eine Dekontaminationsanordnung mit einem speziellen Aufbau bekannt. Dieser umfasst eine kombinierte Zu- und Abluftleitung sowie ein Umluftgebläse, welches nicht wie bei Standarddekontaminationsanordnungen in einem Umlufterzeugerraum oberhalb des zu dekontaminierenden Raums angeordnet ist, sondern in einem Umluftrohr, welches einen unteren Bereich des zu dekontaminierenden Raumes mit einem oberen Bereich des zu dekontaminierenden Raumes verbindet. In der in Fig. 5 der US-Schrift gezeigten Ausführungsform kann der mittels des Gebläses in einem Bereich seitlich des zu dekontaminierenden Isolatorraums erzeugte Umluftstrom gelenkt werden, und zwar in einer ersten Einstellung während der Dekontamination an dem Katalysator getränkten Hepa-Filter vorbei und zu Freispülzwecken durch den Katalysator getränkten Hepa-Filter hindurch. Bei der bekannten Dekontaminationsanordnung wird das Gebläse zur Verteilung des Dekontaminationsmittels benötigt. Die gezeigte Anordnung ist nicht für den Einsatz bei Standarddekontaminationsanordnungen geeignet, bei denen der Umluftstrom nicht über neben dem Isolatorraum verlaufenden Rohrleitungen geführt ist, sondern durch durchsichtige Flachkanäle, die außen von der (durchsichtigen) Isolatorwand begrenzt sind, wie dies in der EP 0 604 925 B1 gezeigt ist.

Zum weiteren Stand der Technik werden die WO 2005/113169 A1, die WO 98/50134 A1 sowie die EP 1 426 122 A1 genannt.

Ausgehend von den zuvor beschriebenen Stand der Technik liegt der Erfindung die Aufgabe zugrunde, eine verbesserte Dekontaminationsanordnung anzugeben, mit welcher das Reduzieren der Dekontaminationsmittelkonzentration in der Raumluft in einer verkürzten Zeitspanne möglich ist.

Diese Aufgabe wird hinsichtlich der Dekontaminationsanordnung mit den Merkmalen des Anspruchs 1 gelöst.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren angegebenen Merkmalen. Zur Vermeidung von Wiederholungen sollen vorrichtungsgemäß offenbarte Merkmale als verfahrensgemäß offenbart gelten und beanspruchbar sein. Ebenso sollen verfahrensgemäß offenbarte Merkmale als vorrichtungsgemäß offenbart gelten und beanspruchbar sein.

Grundsätzlich wäre es möglich, die Zeit, innerhalb derer die Dekontaminationsmittelkonzentration auf das gewünschte Maß reduziert wird, dadurch zu minimieren, dass eine entsprechend größer dimensionierte Frischluftkonditioniereinrichtung vorgesehen wird. Dies wäre jedoch zum einen mit erhöhten Anlagekosten und Platzbedarf für die größere Anlage verbunden und zudem mit erheblich höheren Energiekosten, da eine wesentlich größere Luftmenge konditioniert, d.h. auf die gewünschte Luftfeuchtigkeit getrocknet und auf die gewünschte Temperatur erhitzt oder gekühlt werden müsste. Um diese Nachteile zu vermeiden, schlägt die Erfindung vor, eine katalytisch wirkende Reinigungseinrichtung, wie sie im Stand der Technik häufig in der Abluftleitung angeordnet ist, bei einer erfindungsgemäßen Dekontaminationsanordnung derart zu platzieren, dass - nach erfolgter Dekontamination - die von dem vorhandenen Dekontaminationsmittel zu befreiende Raumluft im Kreislauf durch diese Reinigungseinrichtung mittels eines Gebläses gefördert wird. Es wird also nicht oder zumindest nicht nur mit Frischluft gespült, sondern mit der zunächst noch mit Dekontaminationsmittel belasteten Raumluft selbst. Diese wird vielfach durch die Reinigungseinrichtung gefördert, wodurch die Dekontaminationsmittelkonzentration von Mal zu Mal abnimmt. Bevorzugt wird zum Fördern der Raumluft im Kreislauf ein Gebläse eingesetzt, welches größer (Leistungsstärker) dimensioniert ist als das Frischluftzufuhrgebläse, um hierdurch die Freispülzeit weiter zu reduzieren. Ganz besonders bevorzugt handelt es sich, wie später noch erläutert werden wird, nicht um ein zusätzlich vorzusehendes Gebläse, sondern um ein in vielen Fällen sowieso bereits vorhandenes Umluftgebläse, welches bei Isolatoranwendungen in der Regel derart ausgelegt ist, dass mit diesem Betrieb des Isolators eine gleichmäßige Luftströmungsgeschwindigkeit von 0,45m/s erreicht werden kann. Die Erfindung reduziert also die Freispülzeit ohne die Notwendigkeit, eine größer Dimensionierte Frischluftkonditioniereinrichtung vorsehen zu müssen, da die im Kreislauf geförderte Raumluft ja bereits konditioniert ist. Gegebenenfalls wird während des Freispülens durch Fördern der Raumluft im Kreislauf durch die Reinigungseinrichtung hindurch zusätzlich Frischluft durch eine gegebenenfalls vorgesehene Frischluftzuführleitung zugeführt, insbesondere um hierdurch einen bestimmten geforderten statischen Luftdruck im zu dekontaminierenden Raum, beispielsweise in einem Isolatorraum aufrecht zu erhalten und/oder um die Freispülzeit weiter zu minimieren. Ggf. wird überschüssige Luft durch eine, bei Bedarf zusätzlich mit einer Reinigungseinrichtung versehene Abluftleitung an die Umgebung abgegeben. Auch ist es, insbesondere bei zu dekontaminierenden Schleusen möglich, während des Freispülens auf eine Frischluftzufuhr und/oder eine Raumluftabfuhr in die Umgebung zu verzichten.

Wie eingangs bereits angedeutet, ist vorgesehen, dass es sich bei der Reinigungseinrichtung um einen Katalysator handelt oder dass die Reinigungseinrichtung einen Katalysator umfasst. Für den Fall der Verwendung von Wasserstoffperoxiddampf als Dekontaminationsmittel hat es sich als bevorzugt herausgestellt, als Katalysator MnO₂ oder Palladium einzusetzen, mit welchem das Dekontaminationsmittel chemisch aufspaltbar ist.

Bei Vorrichtungen bzw. zu dekontaminierenden Räumen, beispielsweise bei Schleusen zum Zuführen von Abfüllgut, Pharmazeutika, etc. in einen zu dekontaminierenden Raum ist es erfindungsgemäß vorgesehen, die Reinigungseinrichtung in einen Strömungskanal (Umluftkanal) anzuordnen, vorzugsweise zusammen mit dem Gebläse, wobei der Strömungskanal derart angeordnet ist, dass mit Dekontaminationsmittel versehene Luft aus dem zu dekontaminierenden Raum, insbesondere der Schleuse, angesaugt und die zumindest teilweise bereinigte Luft wieder in den zu dekontaminierenden Raum abgegeben werden kann. Erfindungsgemäß handelt es sich bei dem Strömungskanal bzw. umfasst der Strömungskanal einen Bypass zwischen einer Frischluftleitung zum Zuführen von Frischluft in den zu dekontaminierenden Raum und einer Abluftleitung zum Ablassen von Raumluft an die Umgebung. Erfindungsgemäß ist sowohl die Frischluftleitung als auch die Abluftleitung an jeweils einer Stelle verschließbar, so dass die zu reinigende Raumluft im Kreislauf durch den Bypass förderbar ist, vorzugsweise ohne dass diese Raumluft durch die Frischluftleitung zur Frischluftkonditionseinrichtung und/oder die Abluftleitung in die Umgebung entweichen kann.

Wie eingangs bereits angedeutet, ist es erfindungsgemäß vorgesehen, wenn es sich bei dem zum Einsatz kommenden Gebläse zum Fördern der zu reinigenden Raumluft im Kreislauf durch die Reinigungseinrichtung um ein ohnehin bereits vorhandenes Umluftgebläses zur Erzeugung einer, insbesondere laminaren Luftströmung während des Betriebs des zu dekontaminierenden Raumes handelt. Auf diese Weise muss kein zusätzliches, d.h. separates Gebläse vorgesehen werden, wodurch die Anlagenkosten auf ein Minimum reduziert werden können.

Insbesondere für den Fall, dass die Dekontaminationsanordnung einen Isolator, insbesondere für biotechnische oder pharmatechnische Anwendungen umfasst, ist es bevorzugt, wenn dem vorzugsweise als Umluftgebläse dienenden Gebläse in Strömungsrichtung ein Hochleistungsschwebstofffilter, insbesondere ein sogenannter HEPA-Filter oder ein sogenannter ULPA-Filter, nachgeordnet ist, so dass während des Betriebs der Vorrichtung, insbesondere des Isolators, die im Kreislauf beförderte Raumluft vor dem Einströmen in den zu dekontaminierenden Raum, insbesondere einen Manipulatorraum, von gegebenenfalls noch vorhandenen Schwebstoffen gereinigt wird. Ganz besonders bevorzugt ist es, wenn die, insbesondere katalytisch arbeitende, Reinigungseinrichtung in Strömungsrichtung vor dem Hochleistungsschwebstofffilter angeordnet ist, damit aus Richtung des zu dekontaminierenden Raumes Dekontaminationsmittel (von unten) in den Hochleistungsschwebstofffilter eindiffundieren kann. Grundsätzlich ist es hierzu möglich, die Reinigungseinrichtung in Strömungsrichtung vor dem Gebläse anzuordnen, also derart, dass das Gebläse die Raumluft durch die Reinigungseinrichtung saugt. Ganz besonders bevorzugt ist es jedoch wenn die Reinigungseinrichtung zwischen dem Gebläse und dem Hochleistungsschwebstofffilter angeordnet ist, vorzugsweise in einem Strömungskanal, der aus dem Umlufterzeugerraum ausmündet und der einmündet in den zu dekontaminierenden Raum oder in einen Bereich (Zwischenraum) oberhalb des zu dekontaminierenden Raumes, insbesondere einmündet in einen Bereich oberhalb einer später noch zu erläuternden Membran zur Erzeugung einer laminaren, insbesondere von oben nach unten gerichteten Luftströmung. Besonders bevorzugt ist es, wenn die mindestens eine Zuführleitung zum Zuführen von Dekontaminationsmittel derart angeordnet ist, dass die vorerwähnte Reinigungseinrichtung beim Zuführen des Dekontaminationsmittels gebypasst wird, um zu verhindern, dass frisch hinzugeführtes Dekontaminationsmittel vor Abschluss der Dekontaminationsphase eliminiert wird. Vorzugsweise mündet die mindestens eine Zuführleitung oberhalb der ebenfalls vorerwähnten Membran aus, so dass durch diese das zugeführte gas- und/oder dampfförmige Dekontaminationsmittel gleichmäßig verteilt wird bzw. gleichmäßig verteilt den eigentlichen zu dekontaminierenden Raum erreicht. Ganz besonders bevorzugt ist es, wenn nicht nur eine einzige Zuführleitung vorgesehen ist, sondern wenn mehrere die Reinigungseinrichtung bypassend angeordnete Zuführleitungen zur Zuführung von Dekontaminationsmittel vorgesehen sind. Für den Fall, dass sich herausstellen sollte, dass ein Bereich zwischen der Reinigungseinrichtung und dem Hochleistungsschwebstofffilter nicht ausreichend dekontaminiert werden sollte, ist in Weiterbildung der Erfindung vorgesehen, dass, insbesondere mittels einer Abzweigleitung aus mindestens einer Zuführleitung, Dekontaminationsmittel unmittelbar in einen Bereich zwischen der Reinigungseinrichtung und den Hochleistungsschwebstofffilter zugeführt wird.

Besonders zweckmäßig, insbesondere für die Ausbildung der Dekontaminationsanordnung als Isolator ist es, wenn dem zuvor erwähnten Hochleistungs-schwebstofffilter in Strömungsrichtung eine Membran, beispielsweise ein Siebdruckgewebe, nachgeordnet ist, die sich bevorzugt unmittelbar über dem eigentlichen zu dekontaminierenden Raum, insbesondere einem Manipulatorraum befindet, und die die von dem Umluftgebläse erzeugte Luftströmung in eine laminare Strömung, vorzugsweise mit einer Strömungsgeschwindigkeit von etwa 0,45m/s umwandelt. Ganz besonders bevorzugt ist es, wenn die mindestens eine Zuführleitung zum Zuführen des Dekontaminationsmittels in einem Bereich oberhalb der vorgenannten Membran endet, ganz besonders bevorzugt unter Umgehung, d.h. Bypassung der Reinigungseinrichtung.

Besonders zweckmäßig ist es, wenn die Dekontaminationsanordnung eine Zuführeinrichtung zum Zuführen des gas- und/oder dampfförmigen Dekontaminationsmittels umfasst. Bevorzugt umfasst diese Zuführeinrichtung Mittel zum Erzeugen des gas- und/oder dampfförmigen Dekontaminationsmittels, insbesondere einen, bevorzugt als Blitzverdampfer ausgebildeten, Verdampfer, mit welchem flüssiges Dekontaminationsmittel zur Erzeugung von Dekontaminationsmitteldampf verdampfbar ist. Die Zuführeinrichtung mündet bevorzugt mit einer Zuführleitung in den zu dekontaminierenden Raum und/oder oberhalb einer gegebenenfalls vorgesehenen, zuvor beschriebenen Membran zur Erzeugung einer laminaren Luftströmung. Bevorzugt ist auch eine Abluftleitung vorgesehen, durch die Luft aus dem zu dekontaminierenden Raum, insbesondere an die Umgebung abführbar ist. Besonders zweckmäßig ist es, wenn während der Konditionierphase, während derer der zu dekontaminierende Raum bis zum Erreichen der gewünschten Konzentration mit Dekontaminationsmittel angereichert wird, die Abluftleitung geöffnet ist und Dekontaminationsmittel, insbesondere dampfförmiges Dekontaminationsmittel mit Hilfe von Druckluft aus Drucktanks oder Druckflaschen oder mit Hilfe von konditionierter Frischluft zugeführt wird. Anders ausgedrückt ist es bevorzugt, wenn eine Druckluft- bzw. Frischluftdekontaminationsmittelkonditionierung durchgeführt wird, also nicht Raumluft abgesaugt und angereichert wird wird, um eine Kondensation von Dekontaminationsmittel im zu dekontaminierenden Raum zumindest weitgehend zu verhindern. Bevorzugt wird während der Konditionierphase ein Ventil und/oder ein Abluftgebläse in der Abluftleitung derart geregelt, dass ein gewünschter Luftdruck in den zu dekontaminierenden Raum aufrecht erhalten wird. Bevorzugt erfolgt die Regelung der Abluftmenge zur Einstellung eines geforderten Raumdruckes zusätzlich oder alternativ auch während des bestimmungsgemäßen Betriebs des zu dekontaminierenden Raumes. Bevorzugt ist auch während der auf die Konditionierungsphase folgenden Dekontaminationsphase die Abluftleitung geöffnet und es wird weiterhin mittels Trägerluft Dekontaminationsmittel, gegebenenfalls in geringer Konzentration, zugeführt.

In Weiterbildung der Erfindung ist mit Vorteil vorgesehen, dass die Dekontaminationsvorrichtung eine Frischluftkonditioniereinrichtung zum Konditionieren, d.h. Temperieren und gegebenenfalls Trocknen von Umgebungsluft (Frischluft) aufweist, wobei die Frischluftkonditioniereinrichtung die Frischluft in den zu dekontaminierenden Raum, vorzugsweise über einen Umlufterzeugerraum fördert. Aufgrund der erfindungsgemäßen Anordnung der Reinigungseinrichtung ist es nicht notwendig zur Erzielung kürzerer Freispülzeiten (Dekontaminationsmittelbereinigungszeiten) eine für den eigentlichen Betrieb überdimensionierte Frischluftkonditioniereinrichtung vorzusehen. Bevorzugt ist die Frischluftkonditioniereinrichtung derart ausgelegt, dass mit dieser mittels konditionierter Luft ein 160facher oder geringerfacher Luftwechsel pro Stunde in dem zu dekontaminierenden Raum und sämtlicher unmittelbar strömungstechnisch mit diesem Raum verbundener Räume möglich ist. Bevorzugt ist die Frischluftkonditioniereinrichtung kleiner dimensioniert, so dass maximal ein 100facher oder maximal ein 50facher oder geringerer Luftwechsel pro Stunde möglich ist.

Wie eingangs erwähnt, ist es aus dem Stand der Technik bekannt, in der Abluftleitung (d.h. nicht im Umluftbetrieb) einen MnO₂-Katalysator zum Reinigen der in die Umgebung abzulassenden Raumluft von Wasserstoffperoxid anzuordnen. Dabei ist üblicherweise MnO₂ als Schüttgut in einem Behältnis aufgenommen, das von der Abluft durchströmt werden kann. Nachteilig ist bei dem Schüttgutkatalysator der vergleichsweise hohe Strömungswiderstand, der eine größere Leistung des Gebläses erfordert, um die geforderte Strömungsgeschwindigkeit von 0,45m/s im Betrieb zu gewährleisten. Um den nachteiligen Strömungswiderstand bei maximaler, wirksamer Oberfläche zu minimieren, ist in Weiterbildung der Erfindung mit Vorteil vorgesehen, dass das Katalysatormaterial nicht als Schüttgut vorliegt, sondern als Beschichtung auf einer Trägerstruktur. Bevorzugt ist die Trägerstruktur mit MnO₂ oder Palladium beschichtet. Ganz besonders bevorzugt weist die Trägerstruktur eine möglichst große Oberfläche auf, um möglichst viel wirksame Katalysatoroberfläche auf möglichst geringem Raum unterbringen zu können. Ganz besonders bevorzugt ist es, wenn die Trägerstruktur von einem, insbesondere offenporigen, Metallschaum, insbesondere einem Aluminiumschaum oder einem Nickelschaum gebildet ist, wobei der Metallschaum mit MnO₂ beschichtet ist, insbesondere elektrolytisch. So eignet sich beispielsweise eine Mangansulfatlösung, mit Hilfe derer elektrolytisch ein Mangatdioxidfilm auf die Trägerstruktur, insbesondere die Metallschaumoberfläche, aufbringbar ist. Ganz besonders bevorzugt besteht der Metallschaum aus 50% Nickel, 22% Eisen, 22% Chrom und 6% Aluminium. Alternativ ist es denkbar, den Metallschaum aus 73% Eisen, 22%Chrom und 6% Aluminium auszubilden. Zur Beschichtung des Metallschaums wird dieser bevorzugt als Anode in einem Elektrolytprozess eingesetzt, wobei aus einem Mangansulfatelektrolysebad Mangandioxid an der Anode bei Anlegen einer Spannung abgeschieden wird.

Wie bereits mehrfach angedeutet, ist es besonders bevorzugt, wenn es sich bei dem dekontaminierenden Raum um einen biotechnischen oder pharmatechnischen Manipulatorraum handelt, also einen Raum, in den, insbesondere über fest mit dem Raum verbundene, Handschuhe eingegriffen werden kann. Zusätzlich oder alternativ handelt es sich bei dem zu dekontaminierenden Raum um einen Handhabungsraum für Pharmazeutika und/oder einen Abfüllraum, umfassend eine Abfülleinrichtung zum Abfüllen von Pharmazeutika. Ganz besonders bevorzugt besteht der zu dekontaminierende Raum aus einer Kombination aus einem Manipulatorraum und einem Abfüll- und/oder Handhabungsraum, wobei der Abfüll- und/oder Handhabungsraum bevorzugt unterhalb des Manipulatorraums angeordnet ist, wobei es noch weiter bevorzugt ist, wenn sich oberhalb des Manipulatorraumes ein Umlufterzeugerraum, umfassend ein Umluftgebläse, befindet.

Ganz besonders zweckmäßig ist es, wenn der zu dekontaminierende Raum ein Volumen von mindestens 0,8dm³, vorzugsweise von mindestens 1m³ oder 5m³, ganz besonders bevorzugt von mindestens 10m³, noch weiter bevorzugt von mindestens 20m³ oder mehr aufweist. Denkbar ist es auch, einen ganzen Gebäuderaum mittels der Erfindung zu dekontaminieren bzw. freizuspülen.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: in einem Blockschaltbild den grundsätzlichen Aufbau eines nicht erfindungsgemäßen Ausführungsbeispiels einer erfindungsgemäßen Dekontaminationsanordnung zu Erläuterungszwecken,
- Fig. 2: eine als Isolator ausgebildete nicht erfindungsgemäße Dekontaminationsanordnung zu Erläuterungszwecken, umfassend einen Manipulatorraum als zu dekontaminierenden Raum sowie einen ebenfalls zu dekontaminierenden Umlufterzeugerraum oberhalb des Manipulatorraumes, und
- Fig. 3: in einer schematischen Darstellung den grundsätzlichen Aufbau einer erfindungsgemäßen Dekontaminationsanordnung, umfassend beispielsweise eine Schleuse als zu dekontaminierenden Raum.

In den Figuren sind gleiche Elemente und Elemente mit der gleichen Funktion mit den gleichen Bezugszeichen gekennzeichnet.

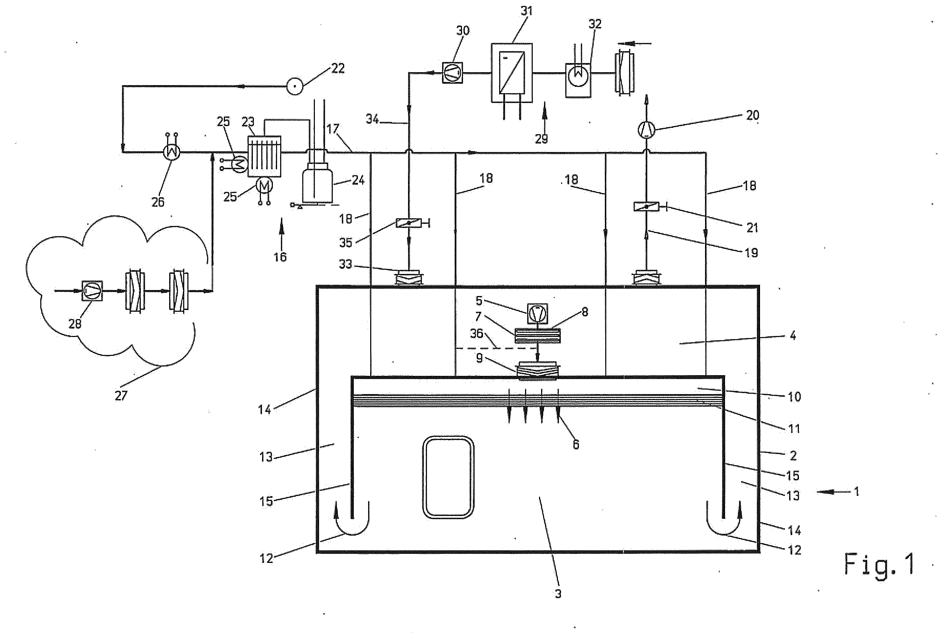

In Fig. 1 ist in einem Blockschaltbild schematisch der Aufbau einer Dekontaminationsanordnung 1 für pharmatechnische Anwendungen gezeigt. Diese umfasst einen Isolator 2 mit einem zu dekontaminierenden Raum 3, der in dem gezeigten Ausführungsbeispiel als Manipulationsraum ausgebildet ist, also mittels Handschuhen von außen durch eine Bedienperson zugänglich ist. Gegebenenfalls kann unterhalb des Manipulatorraums ein Abfüllraum, umfassend eine Abfüllvorrichtung zur Abfüllung von Pharmazeutika vorgesehen sein. Der Manipulatorraum und der Abfüllraum bilden dann eine zu dekontaminierende Raumeinheit (zu dekontaminierender Raum 3).

Oberhalb des zu dekontaminierenden Raumes 3 befindet sich ein Umlufterzeugerraum 4 mit einem als Umluftgebläse dienenden Gebläse 5. Dieses ist derart ausgelegt, dass mit Hilfe des Gebläses 5 eine gleichmäßige Luftströmung 6 von oben nach unten in dem zu dekontaminierenden Raum 3 während des bestimmungsgemäßen Betriebs mit einer Strömungsgeschwindigkeit von etwa 0,45m/s erzeugbar ist. Das Gebläse erzeugt dabei eine turbulente Strömung, die mit einer später noch zu erläuternden Membran, gegebenenfalls in Kombination mit einem Hochleistungsschwebstofffilter in eine laminare Strömung umgewandelt wird.

Dem Gebläse 5 ist in Strömungsrichtung eine Reinigungseinrichtung 7 nachgeordnet. Diese besteht im Wesentlichen aus einem Trägergehäuse für einen Katalysator 8 - in dem gezeigten Ausführungsbeispiel einem MnO₂-Katalysator zur Aufspaltung von Wasserstoffperoxiddampf. Der Katalysator 8 ist gebildet von einem mittels MnO₂ beschichteten Metallschaum, so dass bei Durchströmung des Katalysators 8 ein geringer Strömungswiderstand anfällt und dass der Katalysator 8 möglichst kleinvolumig baut.

Der Reinigungseinrichtung 7 ist in Strömungsrichtung ein Hochleistungsschwebstofffilter 9, beispielsweise ein HEPA-Filter oder ein ULPA-Filter, nachgeordnet. Der von dem Gebläse erzeugte Luftstrom gelangt nach dem Hochleistungsschwebstofffilter 9 in einen Zwischenraum 10, der unten von einer Membran 11, beispielsweise aus einem Siebdruckgewebe, begrenzt ist. Die Membran 11 erzeugt aus dem Luftstrom die laminare Luftströmung 6, welche die vorerwähnte Geschwindigkeit von 0,45m/s im Betrieb aufweist. Die Luft strömt also in dem zu dekontaminierenden Raum 3 nach unten und dort in Pfeilrichtung 12 nach oben in Strömungskanäle 13, die ausgebildet sind zwischen, beispielsweise von Glasplatten gebildeten Isolatorwänden 14 und inneren, parallel zu den Isolatorwänden 14 mit Abstand zu diesen angeordneten transparenten, vorzugsweise aus Glas ausgebildeten, Platten 15. Durch diese Strömungskanäle 13 kann die von dem Gebläse 5 geförderte Raumluft wieder in den Umlufterzeugerraum 4 strömen, aus dem die Luft dann wieder mittels des Gebläses 5 angesaugt wird. Die Raumluft wird also während des Betriebs des Gebläses 5 im Kreislauf durch die Reinigungseinrichtung 7 gefördert.

Um den Isolator 2, genauer den zu kontaminierenden Raum bestimmungsgemäß verwenden bzw. betreiben zu können, muss dieser zunächst dekontaminiert werden. Hierzu ist eine Zuführeinrichtung 16 vorgesehen, mit Hilfe derer gas- und/oder dampfförmiges Dekontaminationsmittel, insbesondere gemischt mit Trägerluft, zugeführt werden kann. Die Zuführeinrichtung 16 umfasst eine zentrale Versorgungsleitung 17, die in dem gezeigten Ausführungsbeispiel als Verteilerleitung dient. Von der Versorgungsleitung 17 gehen in dem gezeigten Ausführungsbeispiel insgesamt vier Zuführleitungen 18 ab, die in den Zwischenraum 10 oberhalb der Membran 11 unter Umgehung der Reinigungseinrichtung 7 ausmünden. Das Dekontaminationsmittel verteilt sich also während der Konditionierungsphase, im Zwischenraum 10, fällt dann nach unten ab durch die Membran 6 hindurch und kann dann in Pfeilrichtung 12 durch die Strömungskanäle 13 nach oben zu einer Abluftleitung 19 strömen, die während der Konditionierungsphase in der der Isolator mit Dekontaminationsmittel angereichert wird geöffnet ist. In der Abluftleitung 19 kann ein Abluftgebläse 20 vorgesehen werden, mit dem gezielt Raumluft aus dem Isolator 2 abgesaugt werden kann. Der Raumluftdruck wird geregelt über das Abluftgebläse 20 in der Abluftleitung 19. Da durch die Abluftleitung 19 Raumluft entweichen kann, verteilt sich das Dekontaminationsmittel im eigentlichen zu dekontaminierenden Raum 3 und auch im Umlufterzeugerraum 4 gleichmäßig. Dadurch, dass während der Konditionierungsphase Raumluft durch die Abluftleitung 19, gegebenenfalls unter Einsatz des Abluftgebläses 20 abgelassen wird, werden Kondensationserscheinungen des Dekontaminationsmittels an den Umfangswänden vermieden.

Die Zuführeinrichtung 16 für Dekontaminationsmittel umfasst in dem gezeigten Ausführungsbeispiel eine Druckluftquelle 22 für Trägerluft, beispielsweise eine Druckluftflasche und einen Verdampfer 23, mit Hilfe dessen Dekontaminationsmittel, hier Wasserstoffperoxid, welches aus einem Vorratsbehälter 24 zugeführt wird, verdampft werden kann. Der Verdampfer 23 verfügt über entsprechende Heizungen 25. Dem Verdampfer 23 ist eine Vorheizung 26 vorgeschaltet, um die Druckluft (Trägerluft) geeignet temperieren zu können.

Die Zuführeinrichtung 16 kann alternativ beispielsweise wie unten dargestellt und wie mit dem Bezugszeichen 27 gekennzeichnet ausgebildet werden. Wesentlicher Unterschied ist hier, dass die Zuführeinrichtung 27 im Gegensatz zu der oben dargestellten Zuführeinrichtung 27 ein Zuführgebläse 28 zum Zuführen von Frischluft aufweist. Diese sollte geeignet konditioniert, d.h. getrocknet und temperiert werden. Der Verdampfer 23 kann auch einer später noch zu erläuternden Frischluftkonditioniereinrichtung nachgeschaltet werden, entweder unmittelbar angeschlossen an die Frischluftleitung oder im Bypass hierzu. Hierdurch kann auf eine separate Konditioniereinrichtung verzichtet werden.

Unabhängig von der Trägerluft (Druckluft oder konditionierte Frischluft) wird jedenfalls zunächst Dekontaminationsmittel während einer Konditionierungsphase, während der die Abluftleitung 19 geöffnet ist, zugeführt. Nach Beendigung der auf die Konditionierungsphase, in der die gewünschte Dekontaminationsmittelkonzentration eingestellt bzw. erreicht wird, folgenden Dekontaminationsphase wird die Zuführeinrichtung 16 bzw. 27 abgeschaltet bzw. abgekoppelt und der zu dekontaminierende Raum 3 wird von Dekontaminationsmittel befreit. Hierzu wird das während der Konditionierungsphase und der Dekontaminierungsphase ausgeschaltete Gebläse 5 angeschaltet, so dass die Raumluft 3 im Kreislauf durch die Reinigungseinrichtung 7 gefördert wird, wodurch die Dekontaminationsmittelkonzentration durch katalytische Wirkung des eingesetzten Katalysators 8 reduziert wird. Nach Erreichen der gewünschten Minimalkonzentration, beispielsweise 1 ppm; 0,5ppm oder weniger kann zum bestimmungsgemäßen Betrieb des zu dekontaminierenden Raums 3 übergegangen werden.

In Fig. 1 ist eine Frischluftkonditionierungseinrichtung 29 zu erkennen, umfassend ein Frischluftgebläse 30, das derart ausgelegt ist, dass mit diesem ein um 150facher Luftwechsel pro Stunde im Isolator 2 möglich ist. Die Frischluft kann mittels einer Kühleinrichtung 31 gekühlt oder mit einer Heizeinrichtung 32 erwärmt werden. Bevorzugt wird die Luft getrocknet. Die zugeführte Frischluft strömt über ein als HEPA- oder ULPA-Filter ausgebildetes Filter 33 in den Umlufterzeugerraum 4. Zu erkennen ist, dass die Frischluftleitung 34, die zum Filter 33 führt, mittels eines Ventils 35 absperrbar ist. Während des bestimmungsgemäßen Gebrauchs wird über die Frischluftkonditioniereinrichtung 29 Frischluft in das System zugeführt, insbesondere um einen unvermeidlichen Druckverlust während des Betriebs, gegebenenfalls vorhandene Leckagestellen in einem gegebenenfalls vorgesehenen Abfüllraum ausgleichen zu können. Leckagestellen sind insbesondere dann unvermeidlich, wenn aus dem zu dekontaminierenden Raum 3 Güter, beispielsweise gefüllte Pharmazeutikabehältnisse, meist kontinuierlich, herausgeführt werden müssen. Der gewünschte Druck im Isolator 1 wird bevorzugt über das Abluftgebläse 20 in der Abluftleitung 19 geregelt.

Es ist nicht notwendig, während der Freispülphase (Umluftphase), also während der Reinigung der Raumluft, Frischluft über die Frischluftleitung 34 zuzuführen. Der Freispülprozess kann jedoch durch diese Maßnahme noch weiter beschleunigt werden.

Um die Strömungsgeschwindigkeit von 0,45m/s zu gewährleisten, ist das als Umluftgebläse während des Betriebs dienende Gebläse größer dimensioniert (insbesondere mindestens doppelt so groß), in dem gezeigten Ausführungsbeispiel fünffach größer d.h. fünffach leistungsstärker dimensioniert als das Frischluftgebläse 30, so dass ein wesentlich größerer Volumenstrom, hier ein fünffach größerer Volumenstrom, durch die Reinigungseinrichtung 7 gefördert werden kann, als Frischluft durch die Frischluftleitung 34 zugeführt werden könnte.

In Fig. 1 ist eine fakultative Stichleitung 36 zu erkennen, die mindestens eine Zuführleitung 18 für Dekontaminationsmittel mit einem Bereich des Strömungskanals zwischen der Reinigungseinrichtung 7 und dem Hochleistungsschwebstofffilter 9 verbindet, um sicherzustellen, dass auch dieser Zwischenbereich ausreichend dekontaminiert wird.

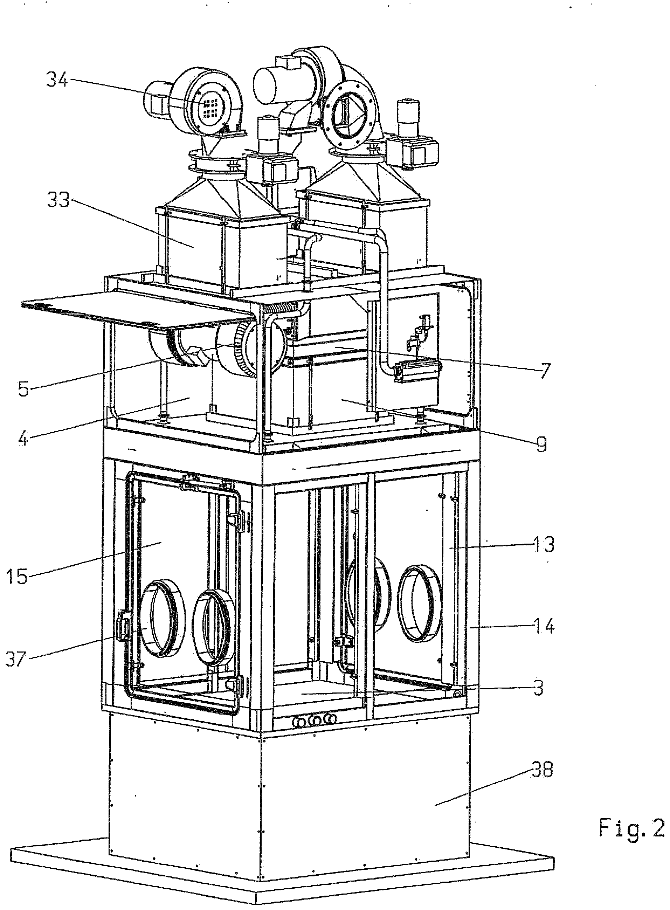

Fig. 2 zeigt einen möglichen Aufbau eines Isolators 2. Zu erkennen ist der als Manipulationsraum ausgebildete, zu dekontaminierende Raum 3, der über Öffnungen 37 von Hand mittels nicht dargestellten Handschuhen zugänglich ist, die am Isolator fixiert sind und die die Öffnungen 37 abdichten. Hinter den aus Glas ausgebildeten Isolatorwänden 14 befinden sich als Glasplatten ausgebildete Platten 15 zur Ausbildung von Strömungskanälen 13, durch die Raumluft aus den zu dekontaminierenden Raum in den oberhalb angeordneten Umlufterzeugerraum 4 strömen kann. In diesen mündet eine Frischluftleitung 34 über ein Filter 33. In dem Umlufterzeugerraum 4 befindet sich das Hochleistungsschwebstofffilter 9, oberhalb dessen die einen MnO₂-Katalysator umfassende Reinigungseinrichtung 7 angeordnet ist und zwar unterhalb eines als Umlufterzeugergebläse ausgebildeten Gebläses 5, welches Raumluft aus dem Umlufterzeugerraum 4 ansaugt und somit im Kreislauf durch die Reinigungseinrichtung 7 fördern kann.

In dem gezeigten Ausführungsbeispiel sitzt der Manipulatorraum auf einem schematisch angedeuteten Gestell 38. Anstelle des Gestells 38 kann ein nach oben offener Abfüllraum, insbesondere beinhaltend eine Abfüllmaschine, oder ein sonstiger Raum vorgesehen werden, der einen Teil des zu dekontaminierenden Raums 3 bildet.

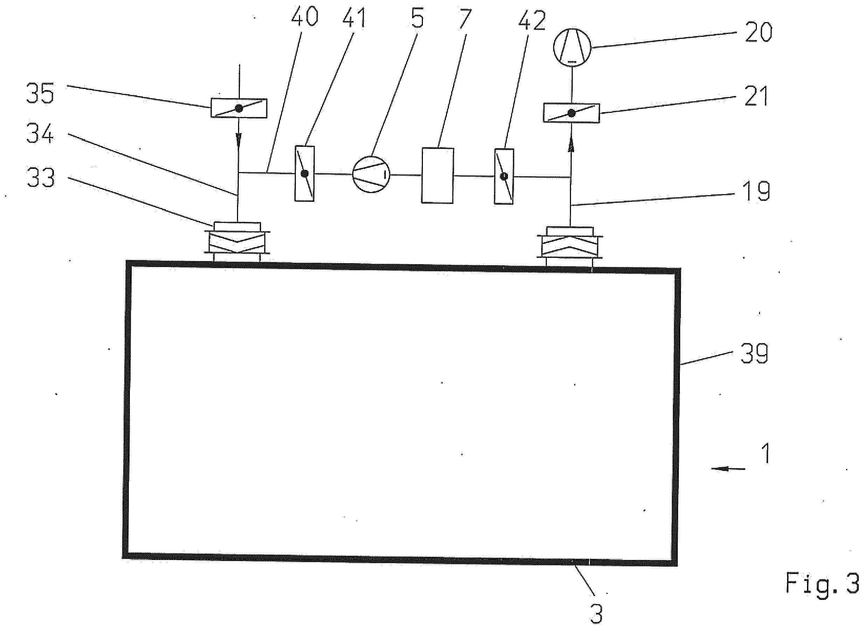

Fig. 3 zeigt in einer schematischen Darstellung den Aufbau einer erfindungsgemäßen Dekontaminationsanordnung 1, die in dem gezeigten Ausführungsbeispiel eine Schleuse 39 als zu dekontaminierenden Raum 3 umfasst. In den zu dekontaminierenden Raum 3 mündet über ein als HEPA- oder ULPA-Filter ausgebildetes Filter 33 eine Frischluftleitung 34, der eine nicht dargestellte Frischluftkonditioniereinrichtung vorgeschaltet ist. Aus dem zu dekontaminierenden Raum 3 mündet eine mittels eines Ventils 21 verschließbare Abluftleitung 19 aus. Dem Filter 33 ist in der Frischluftleitung 34 ein als Absperrventil ausgebildetes Ventil 35 vorgeschaltet. In Strömungsrichtung nach dem Ventil 35 und vor dem Ventil 21 ist die Frischluftleitung 34 über eine als Bypass ausgebildete Umluftleitung 40 verbunden, in der ein Gebläse 5 sowie eine hier katalytisch arbeitende Reinigungseinrichtung 7 angeordnet ist. Die Umluftleitung 40 ist mit je einem Ventil 42, 41 in Strömungsrichtung vor und nach der Reinigungseinrichtung 7 absperrbar. Während einer Dekontaminationsphase sind die Ventile 41, 42 geschlossen und es wird, gegebenenfalls über die Frischluftleitung 34 oder eine separate, nicht dargestellte, Zuführleitung Dekontaminationsmittel zugeführt. Hierbei ist das Ventil 21 geöffnet. Gegebenenfalls wird zusätzlich ein fakultativ vorgesehenes Abluftgebläse 20 betrieben. Nach der auf die Dekontaminationsphase folgenden Reinigungsphase wird zumindest das Ventil 21, bevorzugt auch das Ventil 35, geschlossen, und die Raumluft aus dem zu dekontaminierenden Raum 3 wird im Kreislauf durch die Umluftleitung 40 und damit durch die Reinigungseinrichtung 7 gefördert, um auf diesem Weg Dekontaminationsmittel aus der Raumluft zu entziehen, bevorzugt indem dieses chemisch aufgespaltet wird.

## Patentansprüche

1. Dekontaminationsanordnung, insbesondere für pharmatechnische Anwendungen, umfassend einen zu dekontaminierenden Raum (3), insbesondere einen Isolatorraum sowie eine zum Entziehen von gas- und/oder dampfförmigen Dekontaminationsmittel, insbesondere Wasserstoffperoxid, aus der Raumluft ausgebildete Reinigungseinrichtung (7), wobei die Reinigungseinrichtung (7) derart angeordnet ist, dass die Raumluft durch diese im Kreislauf mittels mindestens eines Gebläses (5) förderbar ist, und wobei die Reinigungseinrichtung (7) einen Katalysator (8), insbesondere MnO₂ oder Palladium, zum chemischen Aufspalten des Dekontaminationsmittels umfasst oder aus einem derartigen Katalysator (8) besteht,
**dadurch gekennzeichnet,**
**dass** die Reinigungseinrichtung (7) in einer als Bypass zwischen einer verschließbaren Frischluftleitung (34) und einer verschließbaren Abluftleitung (19) ausgebildeten Umluftleitung (40) angeordnet ist.

2. Dekontaminationsanordnung nach Anspruch 1,
**dadurch gekennzeichnet,**
**dass** das Gebläse (5) ein Umluftgebläse zur Erzeugung einer Umluftströmung während des Betriebs des zu dekontaminierenden Raumes (3) ist.

3. Dekontaminationsanordnung nach einem der Ansprüche 1 oder 2,
**dadurch gekennzeichnet,**
**dass** dem Gebläse (5) in Strömungsrichtung ein Hochleistungsschwebstofffilter(9), insbesondere einem ULPA-Filter oder einem HEPA-Filter, nachgeordnet ist, und dass die Reinigungseinrichtung (7) in Strömungsrichtung vor dem Hochleistungsschwebstofffilter (9), insbesondere zwischen dem Gebläse (5) und dem Schwebstofffilter angeordnet ist.

4. Dekontaminationsanordnung nach Anspruch 3,
**dadurch gekennzeichnet,**
**dass** dem Hochleistungsschwebstofffilter (9) in Strömungsrichtung eine zum Erzeugen einer laminaren Strömung, insbesondere mit einer Strömungsgeschwindigkeit von etwa 0,45 m/s, ausgebildete Membran (11) nachgeordnet ist und dass Dekontaminationsmittel in einen Bereich oberhalb der Membran (11) zuführbar ist.

5. Dekontaminationsanordnung nach einem der vorhergehenden Ansprüche,
**dadurch gekennzeichnet,**
**dass** eine, vorzugsweise einen Verdampfer (23) umfassende Zuführeinrichtung (16) zum Zuführen des gas- und/oder dampfförmigen Dekontaminationsmittels vorgesehen ist und dass eine Abluftleitung (19) zum Abführen von überschüssigem Dekontaminationsmittel während einer Konditionierphase vorgesehen ist.

6. Dekontaminationsvorrichtung nach einem der vorhergehenden Ansprüche,
**dadurch gekennzeichnet,**
**dass** umfassend eine Frischluftkonditioniereinrichtung (29) zum Konditionieren und Zuführen von Frischluft, wobei die Frischluftkonditioniereinrichtung (29) derart ausgelegt ist, dass mit dieser eine Frischluftvolumenstrom zuführbar ist, um einen weniger als 160fachen oder weniger als 100fachen oder weniger als 50fachen Luftwechsel in dem zu dekontaminierenden Raum (3) und aller strömungstechnisch mit diesem Raum verbundener Räume zu ermöglichen.

7. Dekontaminationsanordnung nach einem der vorhergehenden Ansprüche,
**dadurch gekennzeichnet,**
**dass** die Reinigungseinrichtung (7) eine mit einer reaktiven oder katalytisch wirksamen Substanz, insbesondere MnO₂ oder Palladium, beschichtete Trägerstruktur, insbesondere eine offenporige Metallschaumstruktur oder eine Gitterstruktur umfasst oder daraus besteht.

8. Dekontaminationsanordnung nach Anspruch 7,
**dadurch gekennzeichnet,**
**dass** die Substanz elektrolytisch abgeschieden ist.

9. Dekontaminationsanordnung nach einem der vorhergehenden Ansprüche,
**dadurch gekennzeichnet,**
**dass** der zu dekontaminierende Raum (3) als biologischer oder pharmatechnischer Manipulatorraum oder als Handhabungsraum für Pharamzeutika oder als Abfüllraum mit einer Abfülleinrichtung zum Abfüllen von Pharamzeutika ausgebildet ist oder mindestens einen solchen Raum umfasst.

10. Dekontaminationsanordnung nach einem der vorhergehenden Ansprüche,
**dadurch gekennzeichnet,**
**dass** der zu dekontaminierende Raum (3) ein Volumen von mindestens 0,8dm³, vorzugsweise von mindestens 1m³ oder 5m³, bevorzugt von mindestens 10m³, besonderes bevorzugt von mindestens 20m³ oder mindestens 100m³ aufweist.

## Claims

1. A decontamination system, in particular for use in pharmaceutical technologies, comprising a chamber (3) to be decontaminated, in particular an isolator chamber, as well as a cleaning device (7) for withdrawing gaseous and/or vaporous decontamination medium, in particular hydrogen peroxide, from the ambient air, wherein the cleaning device (7) is disposed in a manner such that the ambient air can be circulated through it by means of at least one fan (5), and wherein the cleaning device (7) comprises a catalyst (8), in particular MnO₂ or palladium, to chemically break down the decontamination medium or consists of a catalyst (8) of this type,
**characterized in that**
the cleaning device (7) is disposed in a circulating air duct (40) constructed as a bypass between a fresh air duct (34) which can be closed off and an exhaust duct (19) which can be closed off.

2. The decontamination system as claimed in claim 1,
**characterized in that**
the fan (5) is a circulating air fan for the production of a circulating air flow during operation of the chamber (3) to be decontaminated.

3. The decontamination system as claimed in claim 1 or claim 2,
**characterized in that**
a high efficiency particulate filter (9), in particular a ULPA filter or a HEPA filter, is disposed downstream of the fan (5) in the direction of flow, and **in that** the cleaning device (7) is disposed upstream of the high efficiency particulate filter (9) in the direction of flow, in particular between the fan (5) and the particulate filter.

4. The decontamination system as claimed in claim 3,
**characterized in that**
a membrane (11) is disposed downstream of the high efficiency particulate filter (9) in the direction of flow for the production of a laminar flow, in particular with a flow rate of approximately 0.45 m/s, and **in that** decontamination medium can be supplied to a zone above the membrane (11).

5. The decontamination system as claimed in one of the preceding claims,
**characterized in that**
a supply device (16), preferably comprising a vaporizer (23), is provided to supply the gaseous and/or vaporous decontamination medium and **in that** an exhaust duct (19) is provided to withdraw surplus decontamination medium during a conditioning phase.

6. The decontamination system as claimed in one of the preceding claims,
**characterized in that**
it comprises a fresh air conditioning device (29) for conditioning and supplying fresh air, wherein the fresh air conditioning device (29) is designed such that it can be used to supply a volume flow of fresh air which can produce an air change of less than 160 times or less than 100 times or less than 50 times in the chamber (3) to be decontaminated and all of the chambers connected with this chamber in terms of the flow technology.

7. The decontamination system as claimed in one of the preceding claims,
**characterized in that**
the cleaning device (7) comprises or consists of a support structure, in particular an open-pore metal foam structure or a grating structure coated with a reactive or catalytically effective substance, in particular MnO₂ or palladium.

8. The decontamination system as claimed in claim 7,
**characterized in that**
the substance has been electrolytically deposited.

9. The decontamination system as claimed in one of the preceding claims,
**characterized in that**
the chamber (3) to be decontaminated is constructed as a manipulation chamber or as an operating chamber for pharmaceuticals or as a filling chamber with a filling device for filling pharmaceuticals, or comprises at least one such chamber.

10. The decontamination system as claimed in one of the preceding claims,
**characterized in that**
the volume of the chamber (3) to be decontaminated is at least 0.8 dm³, preferably at least 1 m³ or 5 m³, preferably at least 10 m³, particularly preferably at least 20 m³ or at least 100 m³.

## Revendications

1. Dispositif de décontamination, en particulier pour des applications pharmaceutiques, comprenant une chambre à décontaminer (3), en particulier une chambre d'isolateur ainsi qu'un dispositif de purification (7) conçu pour extraire un agent de décontamination sous forme de gaz et/ou de vapeur, en particulier du peroxyde d'hydrogène, à partir de l'air de la chambre, dans lequel le dispositif de purification (7) est disposé de telle manière que l'air de la chambre puisse être transporté en circuit à travers celui-ci au moyen d'au moins une soufflante (5), et dans lequel le dispositif de purification (7) comprend un catalyseur (8), en particulier MnO₂ ou palladium, pour la séparation chimique du moyen de décontamination ou se compose d'un tel catalyseur (8), **caractérisé en ce que** le dispositif de purification (7) est disposé dans une conduite de circulation d'air (40), formée comme dérivation entre une conduite d'air frais pouvant être fermée (34) et une conduite d'air sortant pouvant être fermée (19).

2. Dispositif de décontamination selon la revendication 1, **caractérisé en ce que** la soufflante (5) est une soufflante de circulation d'air destinée à produire un courant d'air de circulation pendant le fonctionnement de la chambre à décontaminer (3).

3. Dispositif de décontamination selon l'une quelconque des revendications 1 ou 2, **caractérisé en ce que** la soufflante (5) est suivie, dans la direction d'écoulement, par un filtre pour matières en suspension dans l'air à haut rendement (9), en particulier un filtre ULPA ou un filtre HEPA, et **en ce que** le dispositif de purification (7) est disposé, dans la direction d'écoulement, avant le filtre pour matières en suspension dans l'air à haute puissance (9), en particulier entre la soufflante (5) et le filtre pour matières en suspension dans l'air.

4. Dispositif de décontamination selon la revendication 3, **caractérisé en ce que** le filtre pour matières en suspension dans l'air à haute puissance (9) est suivi, dans la direction d'écoulement, par une membrane (11) conçue pour produire un écoulement laminaire, en particulier avec une vitesse d'écoulement d'environ 0,45 m/s, et **en ce qu'**un moyen de décontamination peut être ajouté dans une région située au-dessus de la membrane (11).

5. Dispositif de décontamination selon l'une quelconque des revendications précédentes, **caractérisé en ce qu'**il est prévu un dispositif d'addition (16), comprenant de préférence un évaporateur (23), pour ajouter l'agent de décontamination sous forme de gaz et/ou de vapeur et **en ce qu'**il est prévu une conduite d'air sortant (19) destinée à évacuer le moyen de décontamination excédentaire pendant une phase de conditionnement.

6. Dispositif de décontamination selon l'une quelconque des revendications précédentes, **caractérisé en ce qu'**il comprend un dispositif de conditionnement d'air frais (29) destiné à conditionner et à ajouter de l'air frais, dans lequel le dispositif de conditionnement d'air frais (29) est conçu de telle manière qu'un courant volumique d'air frais puisse être ajouté avec celui-ci, pour permettre un échange d'air de moins de 160 fois ou de moins de 100 fois ou de moins de 50 fois dans la chambre à décontaminer (3) et toutes les chambres reliées en écoulement à cette chambre.

7. Dispositif de décontamination selon l'une quelconque des revendications précédentes, **caractérisé en ce que** le dispositif de purification (7) comprend ou se compose d'une structure de support revêtue avec une substance réactive ou catalytiquement active, en particulier de MnO₂ ou de palladium, en particulier comprend ou se compose d'une structure de mousse de métal à pores ouverts ou d'une structure de grille.

8. Dispositif de décontamination selon la revendication 7, caractérisé en de que la substance est déposée par voie électrolytique.

9. Dispositif de décontamination selon l'une quelconque des revendications précédentes, **caractérisé en ce que** la chambre à décontaminer (3) se présente sous la forme d'une chambre de manipulateur biologique ou pharmacologique ou d'une chambre de manipulation de produits pharmaceutiques ou d'une chambre de remplissage avec un dispositif de remplissage pour remplir des produits pharmaceutiques ou comprend au moins une telle chambre.

10. Dispositif de décontamination selon l'une quelconque des revendications précédentes, **caractérisé en ce que** la chambre à décontaminer (3) présente un volume d'au moins 0,8 dm³, de préférence d'au moins 1 m³ ou 5 m³, de préférence d'au moins 10 m³, et de préférence encore d'au moins 20 m³ ou d'au moins 100 m³.
